# EUROPEAN PATENT APPLICATION

(11) **EP 4 450 005 A1**
(43) Date of publication of application: **23.10.2024**
(21) Application number: 22923330.9
(22) Date of filing: 11.10.2022
(51) Int. Cl.: A61B 17/14, A61B 17/32

(54) **ULTRASONIC SCALPEL BIT**

(30) Priority: 27.01.2022 CN 202210101478
(71) Applicant: Innolcon Medical Technology (Suzhou) Co., Ltd., Jiangsu 215000 (CN)
(72) Inventor: WANG, Lei, Suzhou, Jiangsu 215000 (CN); LIU, Ke, Suzhou, Jiangsu 215000 (CN); YAN, Zhongyu, Suzhou, Jiangsu 215000 (CN); LUO, Wei, Suzhou, Jiangsu 215000 (CN)
(74) Representative: Doherty, William
(86) International application number: PCT/CN2022/124548
(87) International publication number: WO 2023/142526

(57) **Abstract**

An ultrasonic scalpel bit (100), comprising a bit body (113) in a flat structure, two symmetrical end faces (106) being respectively provided in the thickness direction. At least one side of each end face (106) is provided with a cutting part (131) in the axial direction thereof; the cutting part (131) comprises a surface (132), at least two saw tooth rows (102) being provided on the surface (132), and each saw tooth row (102) being formed by arranging a row of saw teeth (103) in the axial direction of the bit body (113); and a cutting direction of the saw teeth (103) is parallel to the axial direction of the bit body (113), and the saw tooth rows (102) are arranged side by side at intervals; a cooling channel (133) is provided between the adjacent saw tooth rows (102). The cooling channels (133) having a flow guide effect extend from the proximal end of the bit body (113) to a tip part at the distal end, thus guaranteeing that cooling water can be effectively transferred to the tip so as to achieve a good cooling effect on tissue at a deep position. The cooling channels (133) are close to the saw teeth (103) so that heat exchange can be performed quickly.

## Description

### FIELD OF THE INVENTION

The present application relates to the technical field of surgical instrument, in particular, to an ultrasonic scalpel bit (also referred to as ultrasonic scalpel blade).

### BACKGROUND OF THE INVENTION

With rapid development of modern medicine, ultrasonic surgical instruments have been increasingly applied to clinical surgical treatment. They apply ultrasonic energy to surgical operations and have characteristics such as fine cutting, safety, tissue selectivity, low-temperature hemostasis, and so on. They greatly enrich the means of surgical operations, improve the quality of surgical operations, and to some extent, alleviate the sufferings of patients. Among them, the ultrasonic osteotome (or ultrasound bone knife) is an ultrasonic surgical instrument with specific application functions.

The ultrasonic osteotome generally includes a cylindrical body, with a connecting structure provided at its proximal end and a blade provided at its distal end. Currently, there are such kind of ultrasonic scalpel blade as the one disclosed in US patent US8343178 which has a planar bone cutting blade, the one disclosed in US patent USD 667117 which has a distal blade of a sheet form, with both sides of the blade having regular triangular serrated teeth, or an irregularly shaped bone cutting blade as disclosed in European patent EP3586774.

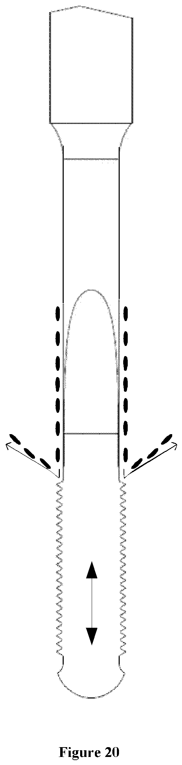

Another inevitable problem related to the tooth shape design of traditional serrated ultrasonic osteotome is that cavitation effect occurs under the action of ultrasound when coolant flows to the nearest serrated tooth surface such that the water flow is atomized and dispersed in a direction perpendicular to the serrated tooth surface, as shown in FIG. 20. Thus, it is difficult for the coolant to flow to the front tooth tip, resulting in a phenomenon where the deeper the cutting thickness is, the greater the caused thermal damage is. Also, the damage caused at the deeper portion is difficult to be observed, which is particularly severe when the tooth tip is completely embedded in the tissue. Currently, the prevailing solution is to increase the flow rate of coolant so as to immerse the entire surface of cut tissue in the coolant, which is a relatively reliable solution. However, it negatively affects the surgical field of vision and the operator's operational accuracy, and it is necessary to use a negative pressure aspirator from time to time to continuously suck the accumulated fluid away, again and again.

US patent US6379371 discloses another cooling method for an ultrasonic scalpel blade, which includes a cooling channel provided inside the blade to directly guide coolant from the blade body to the blade tip. Although, with this structure, the cooling problem may be solved effectively, the processing of the cooling channel inside the ultrasonic scalpel blade is very difficult due to its relatively small structure.

### SUMMARY OF THE INVENTION

An object of the present application is to overcome the shortcomings of the prior art and to provide an ultrasonic scalpel blade with a good cooling effect.

The object of the present application may be achieved through the following technical solutions:
An ultrasonic scalpel blade includes a blade body having a flat structure, with two symmetrical lateral faces provided in a thickness direction thereof, the lateral face, at at least one side thereof, is provided with a cutting portion in an axial direction of the lateral face, the cutting portion includes a surface, on which at least two serrated tooth rows are arranged, each serrated tooth row is formed by aligning a row of serrated teeth in an axial direction of the blade body, a cutting direction of the serrated teeth is parallel to the axial direction of the blade body, the serrated tooth rows are spaced side by side from each other, and a cooling channel is defined between adjacent serrated tooth rows.

Preferably, the cooling channel is located between tooth roots of the adjacent serrated tooth rows.

Preferably, the surface is an axially extending end face of the cutting portion of the blade body between the two symmetrical lateral faces, and the cooling channel is located on the surface.

Preferably, the surface is an axially extending end face of the cutting portion of the blade body between the two symmetrical lateral faces, and the cooling channel is a pathway recessed in the surface.

Preferably, the cooling channel has a depth that is uniform or gradually changing from near to far.

Preferably, an overall width of the cooling channel in an axial direction thereof is uniform.

Preferably, tooth tips of adjacent two serrated tooth rows have the same or opposite orientations.

Preferably, a blade edge arranged at the farthest end of the blade body is at least one of a sharp blade edge, a dull blade edge, or a serrated blade edge.

Preferably, a rake angle γ of the serrated tooth is a positive rake angle, and a tooth tip angle β is not less than 45°.

Preferably, the serrated tooth has a rake angle γ of 15° ± 5° and a clearance angle α of 20° ± 10°.

Preferably, the serrated tooth is a turtle-back tooth, and a gap is formed between adjacent serrated teeth of the same serrated tooth row, and the cooling channel is communicated with the gap.

Preferably, each of the lateral faces of the ultrasonic osteotome is provided with a groove.

Preferably, the blade includes a blade edge located at the farthest end thereof, and two serrated tooth rows symmetrically arranged on both sides thereof along the axis of the blade body.

Preferably, the blade comprises a blade edge located at the farthest end thereof, a serrated tooth row arranged at one side thereof along the axis of the blade body, and a cutting edge located at the symmetrical other side thereof.

The advantageous effects of the present application mainly includes:
The cooling channel providing a flow guiding effect extends from a proximal end of the blade to a distant end of the blade tip, thereby ensuring that coolant can be effectively transferred to the tip so as to achieve a good cooling effect on tissues at a deeper position;
The cooling channel is close to the serrated teeth, thereby allowing for rapid heat exchange;
A hook tooth configuration with two serrated tooth rows arranged reversely ensures that sufficient cutting force is remained during the entire cycle, thereby making cutting efficiency multiply without increasing stress values and reducing a demand on output power of a hardware;
The blade may be well coupled with the load during the cutting process, such that the operator only needs to hold the scalpel for cutting, which improves the comfort during the use of it; and
The blade is provided, at one side thereof, with a serrated tooth row and, at the symmetrical other side, with a cutting edge, therefore the operator can selectively choose the serrated tooth row or the cutting edge for cutting, which expands the applicability of the present application.

### BRIEF DESCRIPTION OF THE DRAWINGS

The following is a further explanation of the technical solutions of the present application in conjunction with the accompanying drawings:
Figure 1 is a schematic diagram of a preferable embodiment of an ultrasonic scalpel blade according to the present application;
Figure 2 is an enlarged view of Part A in Figure 1;
Figure 3 is a front view of Figure 2;
Figure 4 is a top view of Figure 2;
Figure 5 is a schematic diagram showing characteristics of ultrasound used during cutting via a preferable embodiment of an ultrasonic scalpel blade according to the present application;
Figure 6 is a schematic diagram of a second embodiment of an ultrasonic scalpel blade according to the present application;
Figure 7 is a front view of the second embodiment of the ultrasonic scalpel blade according to the present application;
Figure 8 is a comparison schematic diagram of cutting forces for three tooth profiles;
Figure 9 is a comparison schematic diagram of rake angles and cutting forces;
Figure 10 is a comparison schematic diagram of two kinds of tooth-back shapes;
Figure 11 is a schematic diagram showing an arrangement of turtle-back teeth;
Figure 12 is a schematic diagram of a blade of a third embodiment of an ultrasonic osteotome according to the present application;
Figure 13 a schematic diagram of a blade of a fourth embodiment of an ultrasonic osteotome according to the present application;
Figure 14 is a schematic diagram of a blade of a fifth embodiment of an ultrasonic osteotome according to the present application;
Figure 15 is a schematic diagram of the blade of the fifth embodiment of the ultrasonic osteotome according to the present application, viewed in another direction;
Figure 16 is a schematic diagram of a blade of a sixth embodiment of an ultrasonic osteotome according to the present application;
Figure 17 is a schematic diagram of a blade of a seventh embodiment of an ultrasonic osteotome according to the present application;
Figure 18 is a schematic diagram of a blade of an eighth embodiment of an ultrasonic osteotome according to the present application;
Figure 19 is a schematic diagram of a blade of a ninth embodiment of an ultrasonic osteotome according to the present application; and
Figure 20 is a schematic diagram showing an ultrasonic cavitation effect generated by a blade of the prior art.

### DETAILED DESCRIPTION OF THE EMBODIMENTS

Hereinafter, the present application will be described in detail with reference to specific embodiments shown in the accompanying drawings. However, the present application is not limited to these embodiments, and any change on structure, method, or function made by those skilled in the art based on these embodiments is included in the protection scope of the present disclosure.

It should be noted that, in the description of the embodiments, orientations or position relationships indicated by the terms "center", "up", "down", "left", "right", "front", "back", "vertical", "horizontal", "inside", "outside", and so on, are based on orientations or position relationships shown in the drawings, which are used only for the convenience of description and simplification, rather than indicating or implying that the involved device or element must have the specific orientation, and be constructed and operated in the specific orientation, and thus cannot be understood as a limitation of the present application. In addition, the terms "first," "second" and "third" are only used for the purpose of description and cannot be understood as indicating or implying relative importance. Moreover, in the description of the embodiments, with the operator as the reference, a direction close to the operator is referred as a proximal end, and a direction away from the operator is referred as a distal end.

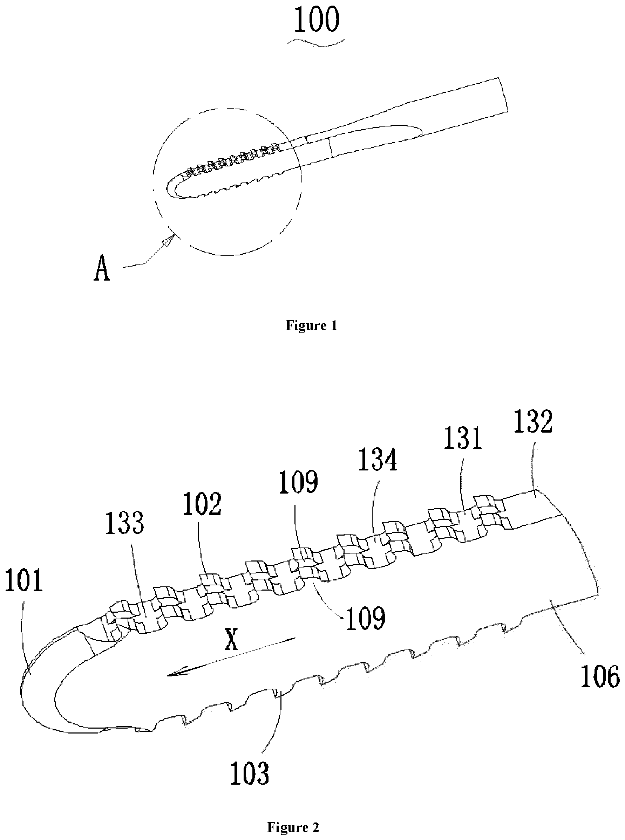

As shown in Figure 1, the present application discloses an ultrasonic scalpel blade 100, as shown in combination with Figure 2, it includes a blade body 113 having a flat structure. The blade body 113 has a blade edge 101 at its farthest end, and has two symmetrical lateral faces 106 in a thickness direction. Each lateral face 106 is provided, at at least one side thereof, with a cutting portion 131 along its axial direction. As is well known to those skilled in the art, the lateral face 106 may be flat, curved, regular or irregular. The lateral face 106 may be provided with the cutting portion 131 at one side, and of course, the lateral face 106 may be provided with the cutting portions 131 at both sides thereof.

Specifically, the cutting portion 131 includes a surface 132. In this preferred embodiment, the surface 132 is an outer axially extending end face of the blade body 113, which is located between the two symmetrical lateral faces 106. At least two serrated tooth rows 102 are arranged on the surface 132. As is well known to those skilled in the art, it is possible to arrange three, four or more serrated tooth rows 102 on the surface 132. Each serrated tooth row 102 is formed by aligning a row of serrated teeth 103 in an axial direction X of the blade body 113. A cutting direction of the serrated teeth 103 is parallel to the axial direction X of the blade body 113, the serrated teeth rows 102 are arranged side by side at intervals, and a cooling channel 133 is formed between adjacent serrated tooth rows 102. In this preferred embodiment, the cooling channel 133 is located between tooth roots 109 of the adjacent serrated tooth rows 102.

As shown in Figure 2, the cooling channel 133 may be located on the surface 132 or may be a pathway (not shown) recessed in the surface 132, if only it is ensured that coolant flowing from a proximal end can pass therethrough and run to its distal end. In the present solution, the cooling channel 133 is arranged between the serrated tooth rows 102 and substantially located at a middle position of the surface 132, so as to avoid ultrasonic cavitation effect of the serrated teeth, thereby allowing the coolant to flow to a distal tip of the blade; and to allow enough coolant to be transferred to the serrated teeth when tips of the serrated teeth are completely embedded into the tissue.

Preferably, an overall width of the cooling channel 133 along its axial direction is uniform. The cooling channel 133 may have a depth that is uniform or gradually changing, for example, gradually increasing from the near to the distant. In this way, the ultrasonic cavitation effect may be utilized better, so as to effectively transfer the coolant to a front end of the serrated teeth, and improve the cooling effect.

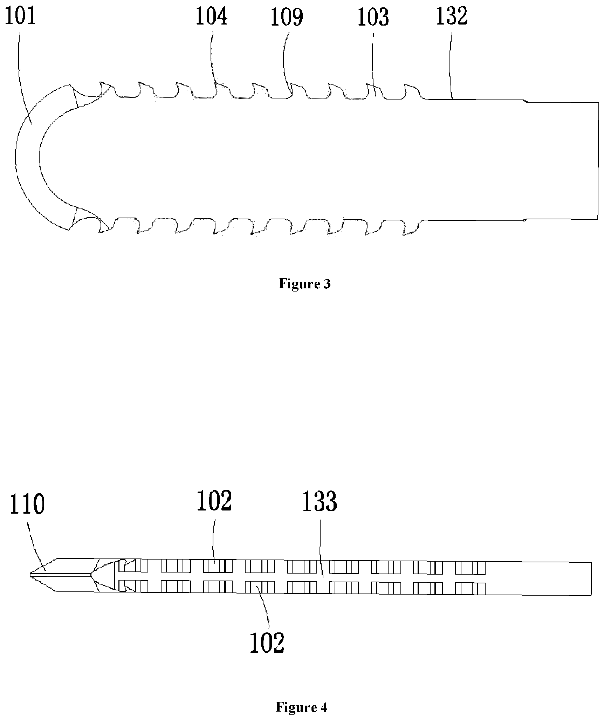

As shown in Figures 3 and 4, in this preferred embodiment, the blade edge 101 has a circular arc projection in the thickness direction of the ultrasonic osteotome, and has a triangular projection 110 in a width direction of the ultrasonic osteotome. With a semi-circle structural design in the thickness direction, the structure is relatively smooth, when the blade edge 101 comes into contact with fragile tissues such as blood vessels and nerves, the structure will not scratch these tissues and the ultrasonic effect will expel these tissues away, also along with sufficient coolant to reduce the heat effect, a good protective effect on these tissues may be obtained. With a triangular design in the width direction a small flat or circular arc surface may be provided at the tip, such that when it comes into contact with cancellous bone and thinner cortical bone, a pointed apex of acute angle may also achieve effective cutting of these tissues.

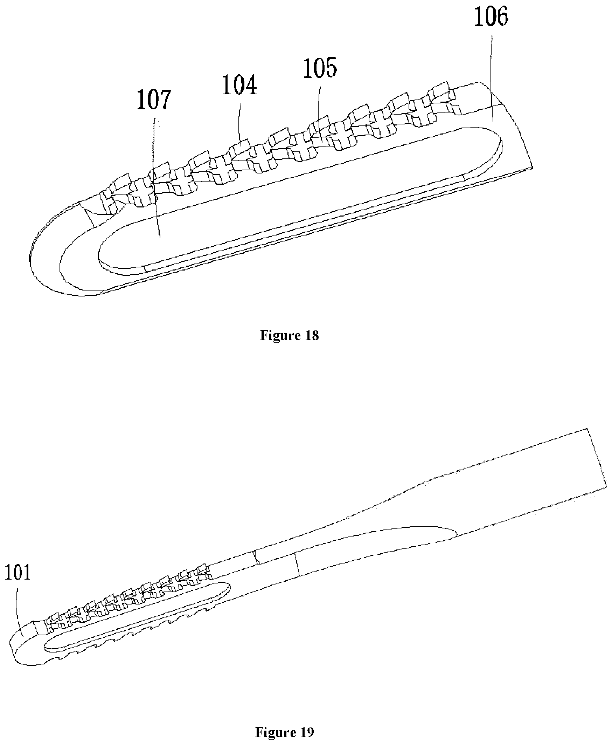

Of course, the blade edge 101 may have other shapes, e.g., in the ninth embodiment as shown in Figure 19, the blade edge 101 is semi-circular and dull-edged. In this way, when ultrasound generated by an ultrasonic generator is transmitted to the blade edge, a stronger expulsion effect is caused, thereby protecting the fragile tissues such as blood vessels and nerves such that they are less prone to be scratched, which can better protect these tissues. Of course, as is well known to those skilled in the art, the blade edge 101 of the blade 113 may also be in other forms. For example, the blade edge 101 is shaped as an extension of the serrated teeth of the serrated tooth row 102, or it may adopt the existing equilateral triangle serrated teeth.

Other structures of the blade body 113 of the present application may be provided based on the shape of the blade edge 101 as mentioned above.

One tooth tip is formed at each of two tip ends of the semicircular arc crown portion of the blade edge 101. The tooth tip is formed through intersection of two circular arc segments, forming a first tooth tip 114. The first tooth tip 114 is composed of an outer circle of R3mm and an inner circle of R1. 5mm of the tip. By making a tangent line along the tooth tip, it can be seen that the tooth has a negative rake angle of -15°, which ensures that the entire blade 101 has a pointed tip with cutting as a secondary consideration and safety as a primary focus, thereby reducing the requirement on operator's operational accuracy and providing better protection to important tissues.

The serrated tooth rows 102 contains two rows arranged side by side, each serrated tooth row 102 is composed of a row of serrated teeth 103. In the preferable embodiment, the serrated teeth 103 of the two serrated tooth rows 102 are exactly the same.

In the preferred embodiment, tooth tips 104 of adjacent two serrated tooth rows 102 have the same orientation, thereby improving a forward cutting force.

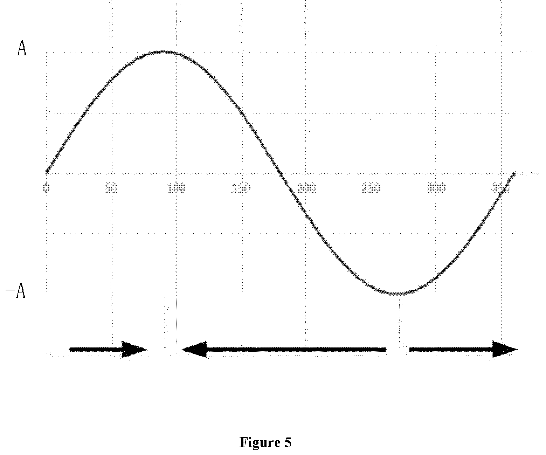

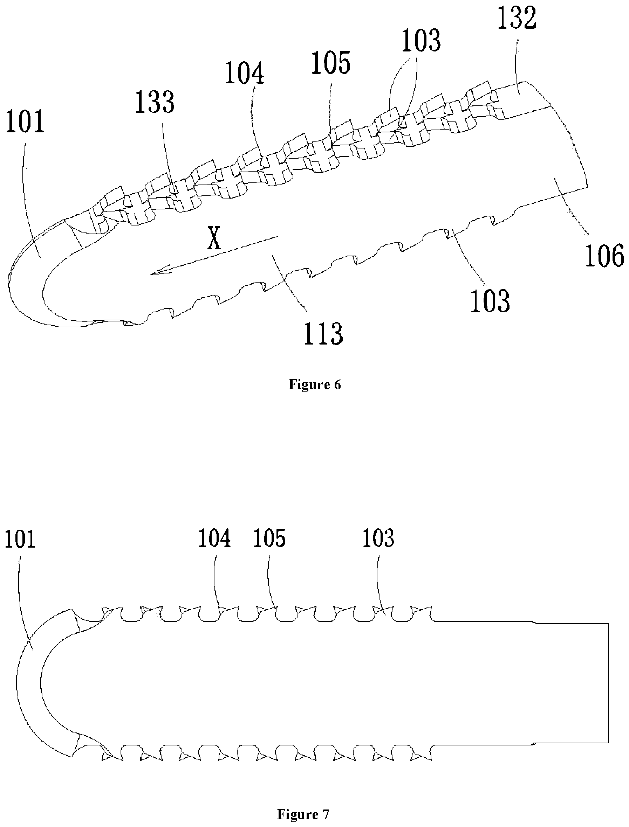

The serrated tooth 103 follows a motion trajectory of ideal simple harmonic vibration, as shown in Figure 5, it starts from an equilibrium position at the zero, and the tooth tip moves towards the distal end of the blade in a positive half cycle and moves towards the proximal end of the blade in a negative half cycle. In order to ensure that the blade has similar cutting effects in both the positive and negative half cycles, in the second embodiment of the present application as shown in Figures 6 and 7, tooth tips 104 and 105 of adjacent two serrated tooth rows 102 have opposite orientations. As it may be well known to those skilled in the art, in a case that there are multiple serrated tooth rows 102, orientations of the tooth tips 104 of the serrated tooth rows 102 may be selectively arranged and combined, alternatively, orientations of the tooth tips 104 of one and the same serrated tooth row 102 may be arranged and combined. All of these equivalent variants fall within the protection scope of the present disclosure without changing the technical purpose.

Three important parameters are involved with respect to the shape of the serrated tooth, including:
γ : rake angle 122, i.e., an angle between a front cutting face and a vertical plane;
α : clearance angle 120, i.e., an angle between a cutting face and a horizontal plane;
β : tooth tip angle 121, i.e., an angle formed between a front cutting face and a rear cutting face.

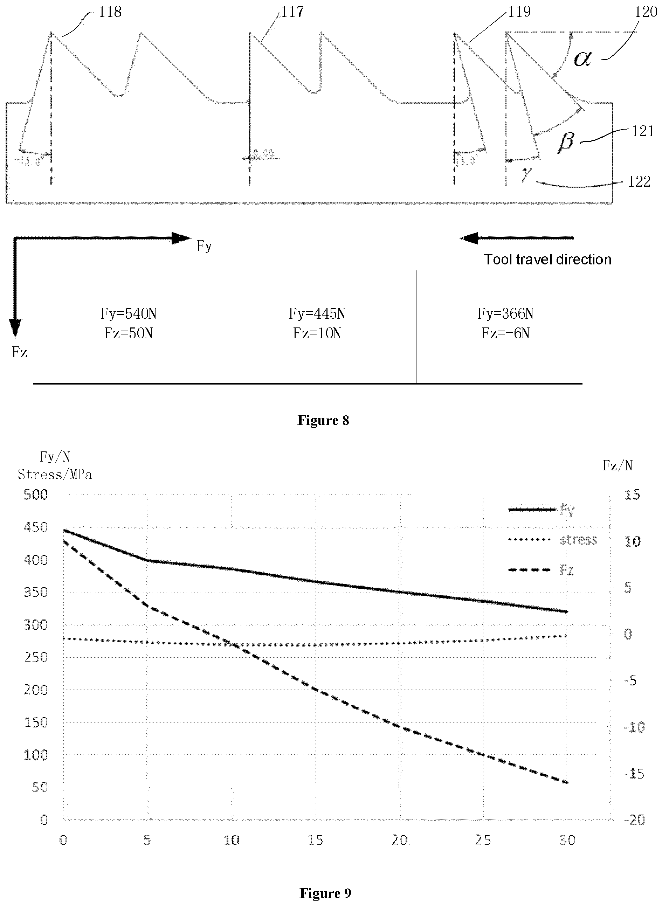

The three kinds of tooth shapes shown in Figure 8 are a standard tooth 117 (with a rake angle of 0°), a blunt tooth 118 (with a rake angle of -15°), and a hook tooth 119 (with a rake angle of +15°), respectively. The traditional serrated tooth has an isosceles triangular shape, which, like the blunt teeth 118, has a negative rake angle γ and belongs to the blunt teeth. Although the same effect may be achieved in both the positive and negative half cycles of movement, the disadvantage is also obvious, i.e., the cutting efficiency is insufficient.

Specifically, referring to Figure 8, an analysis of the cutting force on the scalpel during the cutting is made. It can be seen that, the cutting force on the blunt tooth 118 is the highest, and the cutting force on the hook tooth 119 is the lowest. However, the blunt tooth suffers a significant force Fz deviating from the cutting face, while the hook tooth suffers a negative force Fz deviating from the cutting face. It shows that, when the traditional serrated tooth having the isosceles triangle shape is used, a significant cutting force in travelling is generated, but the blade is prone to be bounced off during the cutting process, and thus it is necessary to provide a sufficient pressure to stabilize the blade. The present application adopts the hook tooth 119, that is, the rake angle γ 122 of the serrated tooth 103 is a positive rake angle. Although the cutting force in travelling is only about 68% of that of the blunt teeth, it is obvious that, the cutting effect of the hook tooth is better under the same load; and the force Fz deviating from the cutting face is negative, which shows that the blade may be well coupled with the load during the cutting, and the operator only needs to hold the scalpel for cutting, which improves the comfort during the use of it.

In order to determine an angle value of the positive rake angle, as shown in Figure 9, the present application provides seven different angle values of the positive rake angle uniformly set from 0° to 30°. It is found that, the cutting force in travelling gradually decreases as the positive rake angle increases, and a negative value of the pressure Fz begins to appear at 10°. Further, as the rake angle increases, a magnitude of the decrease in the cutting force also gradually slows down. Further modal analysis of the blades having the seven kinds of tooth shapes is made. Maximum stress values are statistically analyzed at set working frequencies, and it can be seen that the overall change in stress is relatively small, but as the rake angle increases, the stress value firstly decreases and then increases, with a minimum value appeared at 10° to 20°. Subsequently, as the positive rake angle increases, the maximum stress gradually increases. Moreover, the increase in the positive rake angle will inevitably cause the decrease in the tooth tip angle and the decrease in the strength of the cutting tooth tip, and thus there is a greater likelihood that cutting fatigue would lead to tooth tip fracture. In summary, it is appropriate to set the rake angle between 10° and 20°, and In the present application, the positive rake angle γ 122 is preferably 15°.

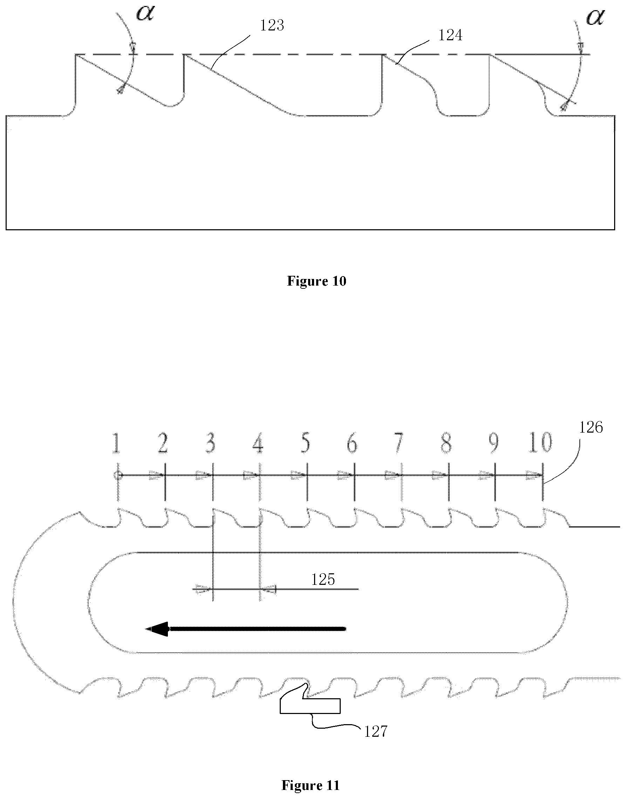

As shown in Figure 10, commonly, there are a straight-back tooth 123 and a turtle-back tooth 124 according to the shape of the tooth back. The present solution employs the turtle-back tooth 124. In cases where the same rake and clearance angles are provided, the turtle-back tooth may have a narrower tooth width, which provides significant advantages in designing of tooth tip strength and tooth spacing. With the same modeling and analyzing methods as mentioned above, an analysis of the angle value of the clearance angle is made. Seven clearance angle parameters uniformly set from 0° to 30° (ignoring the friction effect between the back and the load) are provided to analyze the cutting force. It is found that, as the clearance angle increases, the cutting force in travelling slightly increases. From the perspective of the cutting force alone, a smaller clearance angle will be more favorable. But the smaller the clearance angle is, the easier it is for the tooth back to come into contact with the remaining load tissue, thereby increasing resistance and frictional heat generation. From the perspective of the form, the smaller the clearance angle is, the larger the tooth spacing is, and thus fewer teeth may be arranged within one unit distance, resulting in a decrease in cutting efficiency. Taking all factors into consideration, the rake angle in the present application is preferably 20° ± 10°. The tooth tip angle β 121 of the serrated tooth103 is not less than 45°. The tooth tip angle β 121 of the present application is preferably 55°.

As shown in Figure 11, the turtle-back tooth is designed to have a rake angle of 15° and a clearance angle of 20°. In combination with Figure 2, in order to facilitate shaping, a gap 134 is formed between adjacent teeth 103 in the same serrated tooth raw 102. The cooling channel 133 is communicated with the gap 134.

In the embodiment of the present application, a tooth root fillet of R0.25, a tooth height of 0.6mm, and a shortest tooth spacing of 1.05mm are provided. On the premise of ensuring equal total cutting length as possible, four different tooth spacings are designed according to the following sets of the tooth spacing 125 x teeth number 126: 1.1mm x 13, 1.3mm x 11, 1.5mm x 10, and 1.7mm x 8. Assuming that the total length keeps unchanged, taking a range of 1.3mm to 1.7mm as a reasonable choice, with 1.5mm being a relatively optimal value for the tooth spacing.

The hook tooth structural design of the second embodiment of the present application with two rows arranged reversely (i.e., the tooth tips 104 and 105 of the two serrated tooth rows 102 are oppositely orientated) ensures that there is sufficient cutting force during the entire cutting cycle of cutting hard tissue 127. Through the simulation analysis, the structure provided according to the present application may improve the cutting efficiency exponentially without increasing stress values.

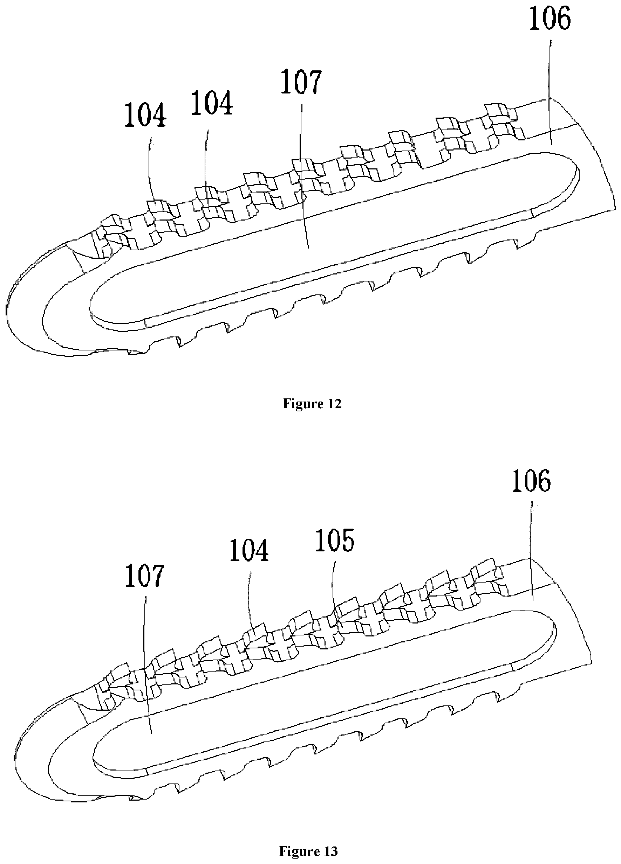

There may be another issue during the cutting of a bone via the ultrasound, when cutting a bone with a large cross-section and a thick tissue, the osteotome blade will inevitably be embedded into the bone for a long time. Nowadays, the prevailing ultrasonic osteotomes are all in a sheet-like structure, with two large-area lateral faces 106 in the thickness direction. When the blade is embedded into the tissue, the planar structure will be in contact with the cut plane for a long time, and under the action of ultrasound, a large amount of heat will be generated, which may easily cause secondary heating of the cut wound and affect bone healing. Also, since it needs that the ultrasonic generator outputs additional energy for such ineffective output, there is a higher demand on output power of the hardware. In view of this, the third embodiment of the present application as shown in Figure 12, differing from the first embodiment, includes a groove 107 on each of the lateral faces 106. In this embodiment, the groove 107 is in the shape of a runway, with the groove on each side having a depth not exceeding 25% of the total thickness, that is, the solid portion left between the grooves having a thickness exceeding 50% of the total thickness, thereby ensuring sufficient mechanical strength. The total length of the groove is greater than the total length of the cutting serrated teeth. In this way, the contact area can be reduced. The fourth embodiment of the present application shown in Figure 13 includes a groove 107 on each of the lateral faces 106 on basis of the hook tooth structure according to the second embodiment which has two serrated tooth rows arranged reversely.

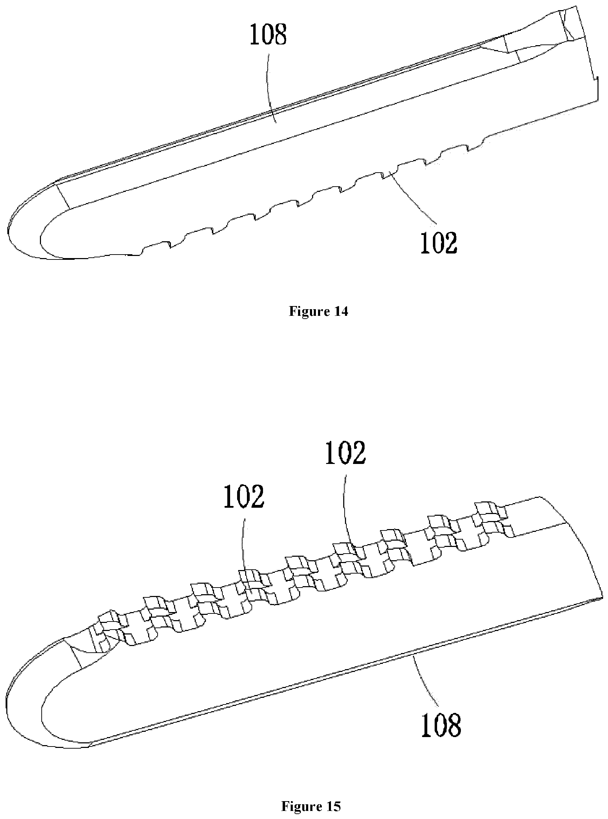

The fifth embodiment of the present application as disclosed in Figures 14 and 15 differs from the preferred embodiment in that the blade 113 is provided, at one side thereof with a serrated tooth row 102 extending along an axis of the blade body, and, at the opposite side thereof, with a cutting edge 108. In this way, the operator may selectively choose the serrated tooth row 102 or the cutting edge 108 for cutting, thereby expanding the usage of the present application.

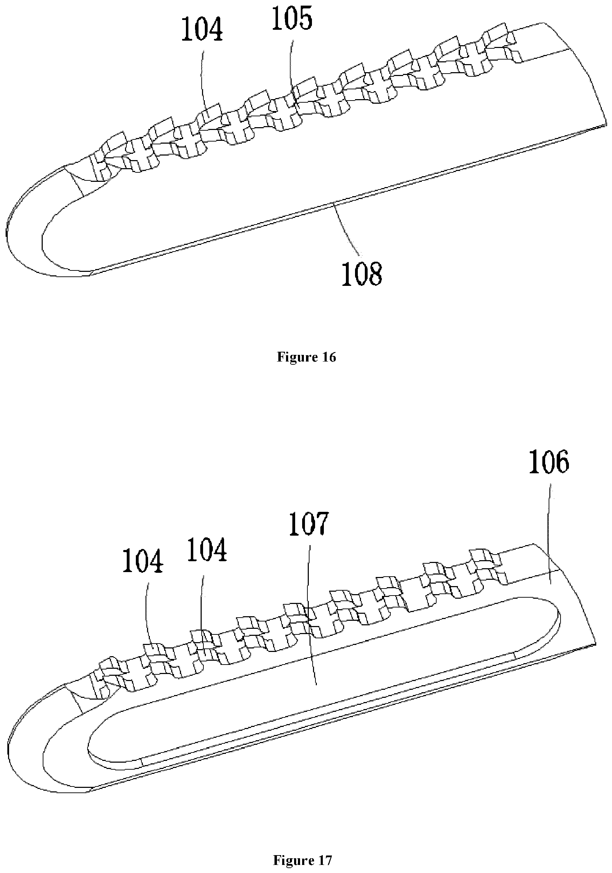

The sixth embodiment of the present application as shown in Figure 16 differs from the fifth embodiment in that the tooth tips 104 and 105 of the two serrated tooth rows 102 have opposite orientations.

The seventh and eighth embodiments of the present application as illustrated in Figures 17 and 18, compared to the fifth and sixth embodiments, have a groove 107 on the lateral face 106. Its effect is similar to that of the second embodiment and will not be repeated here.

It should be understood that, although this specification is described according to individual embodiments, it does not mean that the individual embodiment only includes one independent technical solution. The way in which the specification is described is for clarity only, and those skilled in the art should consider the specification as a whole, and the technical solutions in various embodiments may be appropriately combined to form another embodiment that can be understood by those skilled in the art.

The series of detailed explanations listed above are only specific explanations for some feasible embodiments of the present application, and are not intended to limit the protection scope of the present disclosure. Any equivalent embodiments or alternations that do not deviate from the spirit of the present application should fall within the protection scope of the present application.

## Claims

1. An ultrasonic scalpel blade, **characterized in that** it comprises a blade body (113) having a flat structure, with two symmetrical lateral faces (106) provided in a thickness direction of the blade body, and the lateral face (106) is provided, at at least one side thereof, with a cutting portion (131) in an axial direction of the lateral face, wherein the cutting portion (131) comprises a surface (132), on which at least two serrated tooth rows (102) are arranged, each serrated tooth row (102) is formed by aligning a row of serrated teeth (103) in an axial direction of the blade body (113), a cutting direction of the serrated teeth (103) is parallel to the axial direction of the blade body (113), the serrated tooth rows (102) are spaced side by side from each other, and a cooling channel (133) is defined between adjacent serrated tooth rows (102).

2. The ultrasonic scalpel blade according to claim 1, **characterized in that** the cooling channel (133) is located between tooth roots (109) of the adjacent serrated tooth rows (102).

3. The ultrasonic scalpel blade according to claim 1, **characterized in that** the surface (132) is an axially extending end face of the cutting portion of the blade body (113) and is located between the two symmetrical lateral faces (106), and the cooling channel (133) is located on the surface (132).

4. The ultrasonic scalpel blade according to claim 1, **characterized in that**, the surface (132) is an axially extending end face of the cutting portion of the blade body (113) and is located between the two symmetrical lateral faces (106), and the cooling channel (133) is a pathway recessed in the surface (132).

5. The ultrasonic scalpel blade according to claim 4, **characterized in that** the cooling channel (133) has a depth that is uniform or gradually changing from near to far.

6. The ultrasonic scalpel blade according to claim 1, **characterized in that** an overall width of the cooling channel (133) in an axial direction thereof is uniform.

7. The ultrasonic scalpel blade according to claim 1, **characterized in that** tooth tips (104, 105) of adjacent two serrated tooth rows (102) have the same or opposite orientations.

8. The ultrasonic scalpel blade according to claim 1, **characterized in that** a blade edge (101) arranged at the farthest end of the blade body (113) is at least one of a sharp blade edge, a dull blade edge, or a serrated blade edge.

9. The ultrasonic scalpel blade according to claim 1, **characterized in that** a rake angle γ (122) of the serrated tooth (103) is a positive rake angle, and a tooth tip angle β (121) is not less than 45°.

10. The ultrasonic scalpel blade according to claim 9, **characterized in that** the serrated tooth (103) has the rake angle γ (122) of 15° ± 5° and a clearance angle α (120) of 20° ± 10°.

11. The ultrasonic scalpel blade according to claim 1, **characterized in that** the serrated tooth (103) is a turtle-back tooth, and a gap (134) is formed between adjacent serrated teeth (103) of the same serrated tooth row (102), and the cooling channel (133) is communicated with the gap (134).

12. The ultrasonic scalpel blade according to claim 1, **characterized in that** at least one of the lateral faces (106) of the ultrasonic osteotome is provided with a groove (107).

13. The ultrasonic scalpel blade according to any one of claims 1 to 12, **characterized in that** the blade (113) comprises a blade edge (101) located at the farthest end thereof, and two serrated tooth rows (102) symmetrically arranged at both sides thereof along the axis of the blade body.

14. The ultrasonic scalpel blade according to any one of claims 1 to 12, **characterized in that** the blade (113) comprises a blade edge (101) located at the farthest end of the blade, a serrated tooth row (102) arranged at one side of the blade along the axis of the blade body, and a cutting edge (108) located at the other side of the blade opposite to the one side.
